# EUROPEAN PATENT SPECIFICATION

(11) **EP 1 175 222 B1**
(45) Date of publication and mention of the grant of the patent: **15.12.2004**
(21) Application number: 00923113.5
(22) Date of filing: 19.04.2000
(51) Int. Cl.: A61K 38/14, A61P 31/04

(54) **MONTHLY DOSES OF GLYCOPEPTIDE ANTIBIOTICS FOR LONG-TERM PREVENTION OF STREPTOCOCCUS PNEUMONIAE INFECTIONS**
MONATLICHE GABEN VON GLYKOPEPTID-ANTIBIOTIKA ZUR LANGZEITPRÄVENTION VON STREPTOKOKKUS PNEUMONIAE INFEKTIONEN
DOSES MENSUELLES DE GLYCOPEPTIDES ANTIBIOTIQUES POUR LA PREVENTION DE LONGUE DUREE D'INFECTIONS PAR DES STREPTOCOQUES PNEUMONIAE

(30) Priority: 03.05.1999 US 132258 P
(43) Date of publication of application: 30.01.2002
(73) Proprietor: ELI LILLY AND COMPANY, Indianapolis, Indiana 46285 (US)
(72) Inventor: PARR, Thomas, Reeves, Indianapolis, IN 46236 (US); WASILEWSKI, Margaret, Mary, Indianapolis, IN 46220 (US); ZECKEL, Michel, Lee, Zionsville, IN 46077 (US)
(74) Representative: Srinivasan, Ravi Chandran
(86) International application number: PCT/US2000/008737
(87) International publication number: WO 2000/066144

(56) References cited:
- US-A- 5 840 684
- PATEL ROBIN ET AL: "In vitro activity of LY333328 against vancomycin-resistant enterococci, methicillin-resistant Staphylococcus aureus, and penicillin-resistant Streptococcus pneumoniae." DIAGNOSTIC MICROBIOLOGY AND INFECTIOUS DISEASE, vol. 30, no. 2, February 1998 (1998-02), pages 89-92, XP000961191 ISSN: 0732-8893
- GARCIA-GARROTE FERNANDO ET AL: "In vitro activity of the new glycopeptide LY333328 against multiply resistant gram-positive clinical isolates." ANTIMICROBIAL AGENTS AND CHEMOTHERAPY, vol. 42, no. 9, 1998, pages 2452-2455, XP002153843 ISSN: 0066-4804
- JONES RONALD N ET AL: "In vitro activity and spectrum of LY333328, novel glycopeptide derivative." ANTIMICROBIAL AGENTS AND CHEMOTHERAPY, vol. 41, no. 2, 1997, pages 488-493, XP002153844 ISSN: 0066-4804
- NICAS THALIA I ET AL: "Semisynthetic glycopeptide antibiotics derived from LY264826 active against vancomycin-resistant enterococci." ANTIMICROBIAL AGENTS AND CHEMOTHERAPY, vol. 40, no. 9, 1996, pages 2194-2199, XP002153845 ISSN: 0066-4804

## Description

The present invention relates to the long-term prevention of *Streptococcus pneumoniae* infections with a glycopeptide antibiotic.

Streptococcus pneumoniae is a leading cause of illness and death worldwide. In the United States, infections due to *S. pneumoniae* account for an estimated three thousand cases of meningitis, fifty thousand cases of bacteremia, five hundred thousand cases of pneumonia, and seven million cases of otitis media annually. In addition, *S. pneumoniae* is the most common bacterial cause of respiratory infection, including otitis media, sinusitis, acute exacerbations of chronic bronchitis, and bacterial pneumonia. Although potent antibiotics have been readily available for the treatment of *S*. *pneumoniae* for approximately 50 years, the morbidity and mortality of systemic infection remain substantial, especially among elderly patients and those with underlying diseases. Furthermore, recurrent *S*. *pneumoniae* infection is not uncommon. Current treatments of *S. pneumoniae* suffer from limited efficacy of agents with *in vitro* activity, antimicrobial resistance, and increased susceptibility to infection.

In recent years the incidence of beta-lactam resistant *S. pneumoniae* infection has been increasing worldwide, including Europe, the United States and Asia. In some locales, a significant proportion of resistant *S. pneumoniae* strains are also resistant to macrolides including clarithromycin. Resistance to third generation cephalosporins has also been documented and appears to be increasing. The incidence of isolates highly resistant to penicillin is increasing. For example, in the United States, from 1987 to 1992, the proportion of *S. pneumoniae* strains highly resistant to penicillin increased from 0.02% to 1.3% and in some areas as high as 11.8%. Vancomycin is active *in vitro* and clinically in the treatment of penicillin-resistant *S. pneumoniae* (PRSP) infections; however, clinical failures have been reported. Several reports suggest that β-lactam agents provide appropriate therapy for otitis media, pneumonia, bacteremia and meningitis due to PRSP. However, other data suggests that β-lactam agents may have limited efficacy. With the possible exception of advanced quinolone antibiotics, there are no oral agents with consistent activity against penicillin resistant *S. pneumoniae.*

Certain patient groups are at an increased risk of *S. pneumoniae* infection: for example, (1) patients with hemoglobinopathies (e.g., sickle cell disease); (2) patients who have undergone splenectomy; (3) patients with lymphoma, chronic lymphocytic leukemia and multiple myeloma; (4) patients with HIV infection; and (5) others with various immunodeficiencies. The mortality rate of *S. pneumoniae* sepsis is very high in these patients. Although some infections can be prevented by vaccination, some of these high risk groups fail to develop adequate responses to pneumococcal vaccines. Some patients take chronic penicillin prophylaxis to forestall *S. pneumoniae* infection; however, resistance has been reported in up to 33% to 62% of such patients. (See, i.e., Norris C.F., et al., "Pneumococcal colonization in children with sickle cell disease" *Journal of Pediatrics.* 129(6), 821-7, (1996) ; and Steele R.W., et al, "Colonization with antibiotic-resistant *Streptococcus pneumoniae* in children with sickle cell disease" *Journal of Pediatrics,* 128(4), 531-5, (1996)) Since there are limited oral antibiotic options for treatment or prevention of *S. pneumoniae* infections in vulnerable patients and vaccines may have only limited efficacy, there is a need for improved preventative measures.

Applicants have discovered that the glycopeptide compound N^{DISACC}- (4-(4-chlorophenyl)benzyl)A82846B (also referred to herein as "LY333328") demonstrates (1) significant *in vitro* activity against *S. pneumoniae,* (2) plasma concentrations that exceed the minimum inhibitory concentration (MIC) of penicillin-resistant *S. pneumoniae* for prolonged periods of time following administration; (3) high tissue concentrations for prolonged periods of time following administration; and (4) efficacy in animal models of *S*. *pneumoniae* infection. The combination of all of these properties strongly suggests that N^{DISACC}-(4-(4-chlorophenyl)benzyl) A82846B (or its pharmaceutically acceptable salt, hydrate, or solvate thereof, or a mixture thereof) may be efficacious for long-term prevention of *S. pneumoniae* or prevention of diseases, caused by *S. pneumoniae* infection such as pneumonia, bacteremia, meningitis, septic, arthritis, bronchitis, sinusitis, acute exacerbation's of chronic obstructive lung disease, and otitis media in susceptible individuals.

The present invention provides the use of N^{DISACC}-(4-(4-chlorophenyl) benzyl)A82846B or teicoplanin (or pharmaceutically acceptable salts, hydrates, or solvates thereof) or mixtures thereof in the manufacture of a medicament for the long-term prevention of *S. pneumoniae* infection (or prevention of diseases caused by *S. pneumoniae* infection) in susceptible individuals.

Typically, the medicament is for the periodic administration of an effective dose of N^{DISACC}*-*(4-(4-chlorophenyl)benzyl)A82846B or teicoplanin, or pharmaceutically acceptable salts, hydrates or solvates thereof, or mixtures thereof, wherein the time interval between successive administrations is at least one month.

An effective dose is generally greater than or equal to 0.5 mg/kg body weight per month, preferably from 0.5 to 10 mg/kg, more preferably from 0.5 to 5 mg/kg, even more preferably 0.5 to 3 mg/kg, most preferably from 0.5 to 2.5 mg/kg. In some situations, a dose less than 0.5 mg/kg body weight per month may be effective.

As used herein, the term "susceptible individual" refers to one who is at risk of *S. pneumoniae* infection or at risk of death from *S. pneumoniae* infection. Examples of susceptible individuals having a higher risk for bacterial infection include people with impaired immune function (e.g., immunoglobulin deficiency, splenic dysfunction, splenectomy, HIV infection, impaired leukocyte function, hemoglobinopathies), people with certain malignancies (e.g., multiple myeloma, chronic lympocytic leukemia, lymphoma), people at increased occupation risk (e.g., South African gold miners, welders, painters), people in certain ethnic groups (e.g., American Indians on reservations); people in closed populations during an outbreak of documented *S. pneumoniae* infection (e.g., prisons, military) and others that have immunological deficiences that might enhance their susceptibility to bacterial infection.

The term "dose," "unit dose," "unit dosage," or "effective dose" refers to physically discrete units that contain a predetermined quantity of active ingredient calculated to produce a desired therapeutic effect.

The term "long-term prevention" refers to prevention lasting no less than 28 days.

The term "monthly" refers a frequency of every 28-31 days and "bimonthly" refers a frequency of every 58-62 days.

The term "MIC" or "minimum inhibitory concentration" refers to the lowest concentration of the agent that prevents visible growth after 18 to 24 hours of incubation. "MIC₉₀" refers to the lowest concentration of the agent that prevents 90% growth after 18 to 24 hours of incubation. "MBC" or "minimal bactericidal concentration" refers to the lowest concentration that results in a 99.9% decline in bacterial numbers. Although the value of the MBC as a clinical test has not been established, it may be useful in special instances where very precise knowledge of the ability of a given antimicrobial agent to kill a specific clinical isolate is critical, as in the therapy of bacterial endocarditis.

"N^{DISACC}-(4-(4-chlorophenyl)benzyl)A82846B" or "LY333328" has the following structural formula: 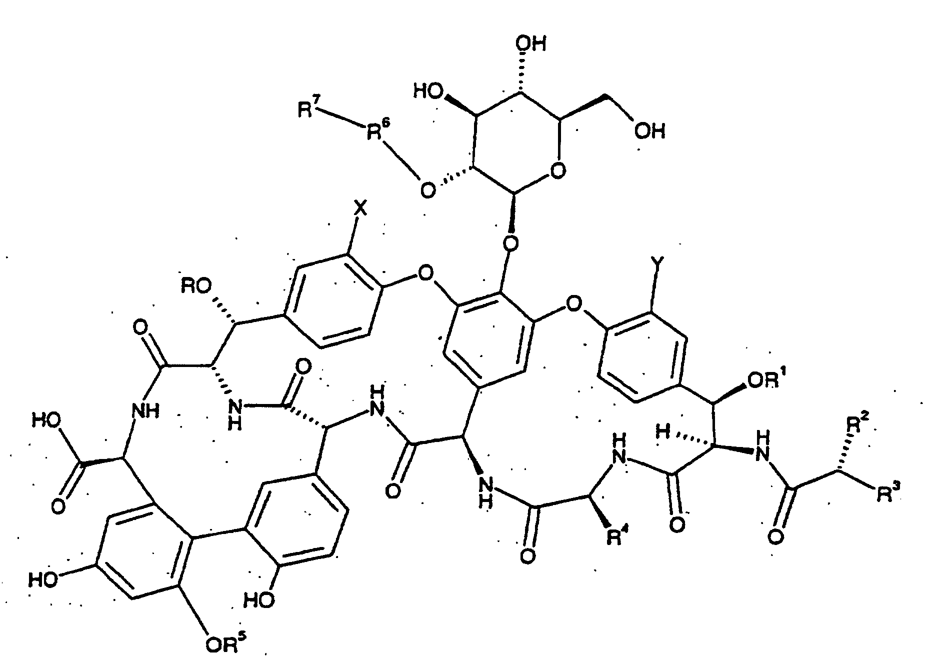 wherein R is 4-epi-vancosaminyl, R¹ is hydrogen, R² is NHCH₃, R³ is CH₂CH(CH₃)₂, R⁴ is CH₂(CO)NH₂, R⁵ is hydrogen, R⁶ is 4-*epi*-vancosaminyl, X and Y are Cl, and R⁷ is 4-(4-chlorophenyl) benzyl (R⁷ is attached to the amino group pendant to the 4-epi-vancosaminyl group). Preparation of N^{DISACC}-(4-(4-chlorophenyl)benzyl)A82845B and analogs thereof may be found in U.S. Patent No. 5,840,684.

"Teicoplanin" refers to a glycopeptide antibiotic complex produced by *Actinoplanes teichomycetius* and is composed of 5 major components that are differentiated by a specific fatty acid moiety ((Z)-4-decanoic acid; 8-methylnonanoic acid; n-decanoic acid; 8-methyldecanoic acid; and 9-methyldecanoic acid). See, i.e., Merck Index Reference No. 9269 - The Merck Index, 12^{th} Edition, Budavari, Susan (Ed), Merck Research Laboratories Division of Merck & Co., Inc., Whitehouse Station, N.J. (1996). Teicoplanin and alkylated derivatives thereof are described in Cooper, et al., U.S. Patent Application No. 09/053848 entitled "Teicoplanin Derivatives filed April 1, 1998.

"NCCLS" refers to the National Committee for Clinical Laboratory Standards located in Wayne, Pennsylvania, USA. The committee recommends and sets performance standards for antimicrobial susceptibility testing.

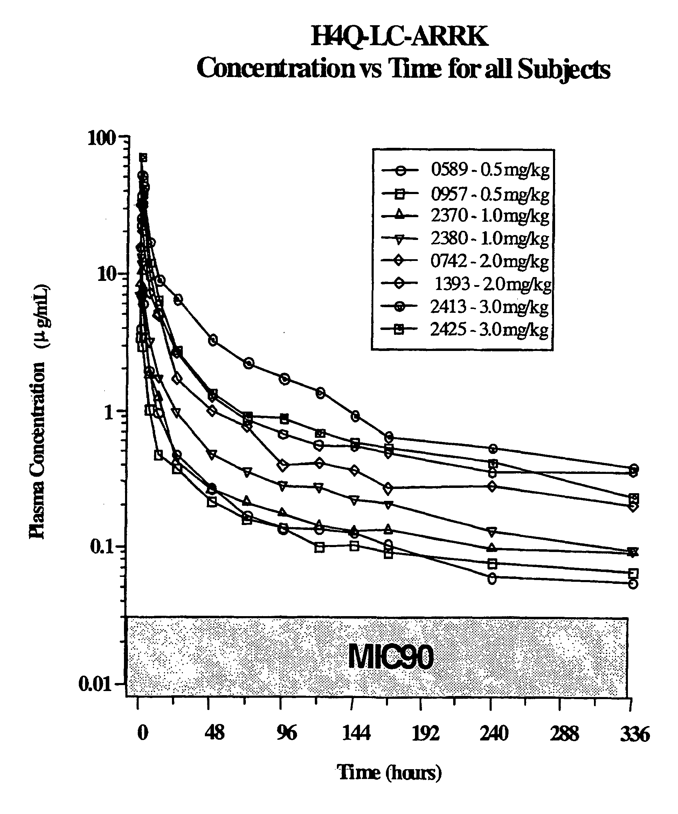

Figure 1 illustrates the relationship between single dose plasma concentrations of N^{DISACC}-(4-(4-chlorophenyl)benzyl)A82846B and MIC₉₀ for penicillin-resistant *S pneumoniae.*

Applicants have discovered that N^{DISACC}-(4-(4-chlorophenyl)benzyl)A82846B has the appropriate properties to allow dosing once a month for treatment against *S. pneumoniae* in susceptible individuals. Although other agents may have prophylactic efficacy, none are expected to work with once a month dosing. Even 1.2 million units of intramusular benzathine penicillin (a drug given once monthly to prevent *Streptococcus pyogenes* infections in patients with a history of rheumatic fever) would not be expected to cover *S. pneumoniae* for more than 10 days at which time the plasma level would fall below 0.06 µg/ml (the MIC₉₀ of penicillin-resistant *S pneumoniae*). Unlike other agents currently known in the art, N^{DISACC}-(4-(4-chlorophenyl)benzyl)A82846B has four properties which strongly suggests that it may be efficacious in long-term prevention of *Streptococcus pneumoniae* in susceptible individuals: (1) significant *in vitro* activity against *S. pneumoniae;* (2) plasma concentrations that exceed the MIC of penicillin-resistant *S. pneumoniae* for prolonged periods of time; (3) high tissue concentrations for prolonged periods of time; and (4) efficacy in animal models of *S. pneumoniae* infection.

### In Vitro Activity of N^{D}^{ISACC}- (4-(4-chlorophenyl)benzyl)A82846B Against Streptococcus pneumoniae:

Table 1 lists the in vitro activity of N^{DISACC}-(4-(4-chlorophenyl)benzyl)A82846B and other compounds against a worldwide collection of *Streptococcus pneumoniae.* N^{DISACC}-(4-(4-chlorophenyl)benzyl)A82846B clearly demonstates a significant improvement against all three strains. Although not as active as N^{DISACC}-(4-(4-chlorophenyl)benzyl)A82846B, Teicoplanin also demonstrates a notable improvement.

**Table 1**

| Comparative MIC₉₀ of Various Antibiotics Against *Streptococcus pneumoniae* | | | |
|---|---|---|---|
| | MIC₉₀ (µg/mL) | | |
| | Sensitive N = 50 | Intermediate N = 40 | Resistant N = 42 |
| Penicillin G | 0.06 | 1.0 | 4.0 |
| N^{DISACC}-(4-(4-chlorophenyl) benzyl)A82846B | 0.008 | 0.015 | 0.03 |
| Vancomycin | 0.5 | 0.5 | 0.5 |
| Teicoplanin | 0.06 | 0.06 | 0.06 |
| Ceftriaxone | 0.06 | 0.5 | 2.0 |
| Rifampin | 0.03 | 0.015 | > 8.0 |
| Imipenem | 0.03 | 0.125 | 0.5 |

MICs were determined using NCCLS broth microdilution methodology (see, Fasola E., Spangler SK., Ednie LM., Jacobs MR., Bajaksouzian S., and Appelbaum PC., "Comparative activities of LY 333328 against penicillin-susceptible and -resistant pneumococci" *Antimicrobial Agents & Chemotherapy,* 40(11), 2661-3 (1996)). Sensitive, intermediate, and resistant are defined using NCCLS criteria: sensitive = MIC < 0.012 µg/mL of penicillin; intermediate = MIC ≥ 0.012 and MIC < 2.0 µg/mL of penicillin; and resistant = MIC ≥ 2 µg/mL.

### Plasma Concentrations Exceed the MIC of S. pneumoniae for Prolonged Time Period:

The figure in the drawings illustrates the relationship between single dose plasma concentrations of N^{DISACC}-(4-(4-chlorophenyl)- benzyl) A82846B and MIC₉₀ for penicillin-resistant *S. pneumoniae.* The data supports that a single dose of 1 to 3 mg/kg body weight should provide plasma concentrations in excess of the MIC₉₀ of penicillin-resistant S pneumoniae for 4 to 6 weeks.

Although single doses of N^{DISACC}-(4-(4-chlorophenyl)benzyl)A82846B may provide high plasma concentrations for a prolonged period of time, it may also be highly desirable to maintain high concentrations in respiratory tissues (the main portal of initial tissue infection and invasion). Following a 5 mg/kg body weight dose of ¹⁴C- N^{DISACC}- (4-(4-chlorophenyl)benzyl)A82846B in rats, lung tissue half life proved to be 29 days.

### Efficacy of N^{DISACC}-(4-(4-chlorophenyl)benzyl)A82846B in Animal Models of S. pneumoniae Infection:

N^{DISACC}- (4-(4-chlorophenyl) benzyl)A828468 shows significant activity in *in vivo* models. As listed in Table 2, N^{DISACC}-(4-(4-chlorophenyl)benzyl)A82846B appeared to have activity 30 to 40 times that of vancomycin when given subcutaneously to mice with systemic streptococcal or staphylococcal infections. This marked effect was present even though N^{DISACC}-(4-(4-chlorophenyl) benzyl)A82846B is absorbed poorly following subcutaneous administration in mice. In a murine model of systemic infection, various dosing regimens of N^{DISACC}-(4-(4-chlorophenyl)benzyl)A82846B administered subcutaneously were compared in animals inoculated intraperitoneally with either *S pneumoniae* or *S* aureus. The total dose ranged from 0.18 to 2.01 mg/kg body weight, subdivided into 1 to 24 doses during a 48-hour treatment period. Pharmacokinetic studies were performed in uninfected animals using a bioassay. Using multiple regression analysis to find the pharmacodynamic parameter associated with survival, time above the MIC appeared-to be more important (p<0.00001) than the maximum plasma concentration Cₘₐₓ (p=0.01); however, for 1- and 2-dose regimens near the ED₅₀, Cₘₐₓ appeared to be the best predictor of outcome. A more detailed description of this study may be found in Knudsen, J., et al., "Pharmacodynamics of glycopeptides in Animal Models" Abstracts of the 20^{th} International Congress of Chemotherapy, Abstract No. 4076, June (1997). In a similar neutropenic model of *S. pneumoniae* bacteremia in mice, N^{DISACC}- (4-(4-chlorophenyl) benzyl)A82846B administered subcutaneously or intravenously at doses ≥ 5 mg/kg body weight eliminated bacteremia.

**Table 2**

| Efficacy in a Mouse Protection Model | | | |
|---|---|---|---|
| | ED₅₀a (mg/kg/dose) | | |
| | *S pneumoniae* Park I | *S pyogenes* C-203 | *S aureus* SA027 |
| Vancomycin | 1-1 | 0.80 | 0.70 |
| A82846B | 0.18 | 0.18 | not tested |
| N^{DISACC}-(4-(4-chlorophenyl) benzyl)A82846B | 0.028 | 0.045 | 0.009 |

| | | | |
|---|---|---|---|
| ^{a}ED₅₀. dose effective in protecting 50% of mice from lethal infection. Infection was established by intraperitoneal bacterial challenge: treatment was two subcutaneous doses 1 and 5 hours after challenge. | | | |

### Bactericidal activity of N^{DISACC}-(4-(4-chlorophenyl)benzyl) A82846B against Streptococcus pneumoniae in an in-vivo model of S. pneumoniae meningitis:

NZW Rabbits were infected intracisternally with 10⁶ CFU of a penicillin-sensitive *S. pneumoniae* type 3 strain (MIC for N^{DISACC}-(4-(4-chlorophenyl)benzyl)A82846B = 0.015 mg/l, MBC 0.03 mg/l). At 12 h after infection, they were treated with a single dose of N^{DISACC}-(4-(4-chlorophenyl)benzyl)A82846B [1 mg/kg (n = 5), 2.5 mg/kg (n = 5), 10 mg/kg (n = 10), 40 g/kg (n = 2)] . N^{DISACC}-(4-(4-chlorophenyl)benzyl)A82846B was dissolved in 5% glucose and infused over 30 min. Controls received a bolus of ceftriaxone (20 mg/kg body weight) followed by a continuous infusion of 10 mg/kg/h (n = 12). The cerebrospinal fluid (CSF) was drawn at 12, 14, 17, 20, and 24h for the determination of bacterial titers, leukocyte densities, lipoteichoic/teichoic acid, lactate and protein concentrations. The concentration of the neuron-specific enolase in CSF was measured at 24h. Bactericidal activity in CSF was estimated by log-linear regression of bacterial titers versus time. Additionally, time-kill curves were performed with 10 mg/l N^{DISACC}-(4-(4-chlorophenyl)benzyl)A82846B.

*In* vitro, 10 mg/l of N^{DISACC}-(4-(4-chlorophenyl)benzyl) A82846B killed *S.pneumoniae* within 1h, whereas cultures treated with 10 mg/l ceftriaxone were sterile after 12h. A single dose of 10 mg/kg body weight of N^{DISACC}-(4-(4-chlorophenyl)benzyl)A82846B reduced the CSF bacterial titers as rapidly as ceftriaxone 10 mg/kg/h (Δlog CFU/ml/h ± SD: -0.29 ± 0.21 vs. -0.33 ± 0.15). Protein, lactate and lipoteichoic/teichoic acid concentrations in CSF showed no significant differences. The bactericidal activity of 2.5 mg/kg N^{DISACC}-(4-(4-chlorophenyl)benzyl)A82846B was slightly lower (Δlog CFU/ml/h ± SD: -0.26 ± 0.22). A dose of 40 mg/kg N^{DISACC}-(4-(4-chlorophenyl)benzyl)A82846B reduced the bacterial titers by -0.52 ± 0.02 Δlog CFU/ml/h. A dose of 1 mg/kg body weight was only bacteriostatic (Δlog CFU/ml/h ± SD: 0.01 ± 0.11). The half-maximal dose (K_{D}) and the maximum bactericidal rate (Eₘₐₓ) of LY333328 as estimated by Lineweaver-Burk plot were 5.3 mg/kg body weight and -0.88 Δlog CFU/ml/h, respectively. The mean concentration of the neuron-specific enolase in CSF at 24h as a parameter of neuronal damage was slightly lower in N^{DISACC}-(4-(4-chlorophenyl)benzyl)A82846B treated animals (10 mg/kg body weight) than in rabbits receiving ceftriaxone (92.3 ± 68.7 vs. 152.5 t 97; p = 0.14).

The *in-vivo* activity suggests that N^{DISACC}- (4-(4-chlorophenyl)benzyl)A82846B may be useful for the treatment of *S.pneumoniae* meningitis. The inflammatory reaction during treatment with N^{DISACC}-(4-(4-chlorophenyl)benzyl)A82846B was comparable to that during ceftriaxone therapy.

### Safety and Pharmacokinetics of Single Intravenous Doses of N^{DISACC}-(4-(4-chlorophenyl)benzyl)A82846B Diphosphate in Eight Healthy Men:

An open-label, uncontrolled, dose-escalation study was conducted with eight healthy men. All subjects were between 22 and 50 years of age and within 10% of their ideal body weights. Single does, ranging from 0.5 mg/kg body weight to 3 mg/kg body weight, were administered intravenously over 30 minutes. Plasma samples were collected just prior to the start of infusion and at intervals for 336 hours after the end of the infusion for assessment of drug concentration, biochemical and hematologic parameters. N^{DISACC}-(4-(4-chlorophenyl)benzyl) A82846B plasma and urine concentrations were determined by HPLC and competitive binding radioimmunoassay, respectively. Individual plasma concentration-time profiles were evaluated based on compartmental analysis techniques. The results are summarized in Table 3 below.

**Table 3**

| **Parameter*** | **Mean (%CV)** | **Range** |
|---|---|---|
| AUC_{0-∞} (µg hr/ml)/(mg/kg) | 201 (62) | 120 to 305 |
| Cₘₐₓ (µg/ml)/(mg/kg) | 16.5 (20) | 13.1 to 23.6 |
| Clₚ (ml/min/kg) | 0.0896 (30) | 0.0547 to 0.138 |
| Vₛₛ (1/kg) | 1.08 (45) | 0.650 to 1.92 |
| T_{1/2} (hr) | 251 (96) | 132 to 356 |
| % dose excreted in urine | 3.1 (42) | 1.51 to 5.62 |

| | | |
|---|---|---|
| * AUC_{0-∞} and Cₘₐₓ are normalized to dose and weight (unit per mg/kg dose). | | |

At the end of the infusion, plasma concentrations of N^{DISACC}*-*(4-(4-chlorophenyl)benzyl)A82846B followed a tri-exponential decline. The maximum plasma concentration (Cₘₐₓ) and the area under the curve (AUC_{0-∞}) appeared to increase linearly and proportionally with dose, within the dose range studied. Plasma concentrations normalized to dose and body weight were generally consistent across the various doses. Systematic change in plasma clearance (Clₚ), steady-state volume of distribution (Vₛₛ), and half-life (t_{1/2}) were not observed over the dose range studied. The terminal t_{1/2} of N^{DISACC}-(4-(4-chlorophenyl)benzyl)A82846B was evaluated from plasma data collected in less than two t_{1/2} in most subjects and consequently the pharmacokinetic results should be interpreted cautiously. The area under the terminal phase represented approximately 50% of total AUC_{0-∞} N^{DISACC}-(4-(4-chlorophenyl) benzyl)A82846B has unique pharmacologic properties, including an unusually long terminal t_{1/2} (10.5 days). The safety data collected and all adverse events noted indicate the drug was well tolerated and safe at these single doses.

Preclinical microbiology, ADME, and animal models as well as initial single dose pharmacokinetic studies in volunteers suggest that N^{DISACC}- (4-(4-chlorophenyl)-benzyl)A82846B may provide important benefits for patients at increased risk of serious *Streptococcus pneumoniae* infection. Single intravenous doses of 0.5 mg/kg (or lower) may provide curative therapy for infections caused by *S. pneumoniae,* including pneumonia, bacteremia, meningitis, septic arthritis, bronchitis, sinusitis, acute exacerbation's of chronic obstructive lung disease, and otitis media. In the case of recurrent *S. pneumoniae* infections (recurrent otitis media, recurrent or chronic sinusitis, chronic obstructive lung disease), a single dose may reduce the frequency or severity of relapse within 60 days of administering N^{DISACC}-(4-(4-chlorophenyl)benzyl)A82846B. More importantly, these data suggest that N^{DISACC}-(4-(4-chlorophenyl)benzyl)A82846B may provide protection through infrequent administration, such as every 4 to 8 weeks (monthly or bimonthly).

### Preparation of N^{DISACC}-(4-(4-chlorophenyl)benzyl)A82846B:

A three liter 3-necked flask is fitted with a condenser, nitrogen inlet and overhead mechanical stirring apparatus. The flask is charged with pulverized A82846B acetate salt (20.0 g, 1.21 x 10⁻³ mol) and methanol (1000 mL) under a nitrogen atmosphere. 4'-chlorobiphenyl carboxaldehyde (2.88 g, 1.33 x 10⁻² mol, 1.1 eq.) is added to this stirred mixture, followed by methanol (500 mL). Finally, sodium cyanoborohydride (0.84 g, 1.33 x 10⁻² mol, 1.1 eq.) is added followed by methanol (500 mL). The resulting mixture is heated to reflux (about 65°C).

After 1 hour at reflux, the reaction mixture attained homogeneity. After 25 hours at reflux, the heat source is removed and the clear reaction mixture is measured with a pH meter (6.97 at 58.0°C). 1 N NaOH (22.8 mL) is added dropwise to adjust the pH to 9.0 (at 54.7°C). The flask is equipped with a distillation head and the mixture is concentrated under partial vacuum to a weight of 322.3 grams while maintaining the pot temperature between 40-45°C.

The distillation head is replaced with an addition funnel containing 500 mL of isopropanol (IPA). The IPA is added dropwise to the room temperature solution over 1 hour. After approximately 1/3 of the IPA is added, a granular precipitate formed. The remaining IPA is added at a faster rate after precipitation had commenced. The flask is weighed (714.4 grams of the IPA/methanol slurry).

The flask is re-equipped with a still-head and distilled under partial vacuum to remove the remaining methanol. The resulting slurry (377.8 g) is allowed to chill in the freezer overnight. The crude product is filtered through a polypropylene pad and rinsed twice with 25 mL of cold IPA. After pulling dry on the funnel for 5 minutes, the material is placed in the vacuum oven to dry at 40°C. A light pink solid (22.87 g (theory = 22.43 g)) is recovered. HPLC analysis versus a standard indicated 68.0% weight percent of N^{DISACC}-(4-(4-chlorophenyl)benzyl)-A82846B in the crude solid, which translates into a corrected crude yield of 69.3%.

The products of the reaction are generally analyzed by reverse-phase HPLC utilizing a Zorbax™ SB-C18 column with ultraviolet light (UV; 230 nm) detection. A 20 minute gradient solvent system consisting of 95% aqueous buffer/5% CH₃CN at time=0 minutes to 40% aqueous buffer/60% CH₃CN at time=20 minutes is used, where the aqueous buffer is TEAP (5 ml CH₃CN, 3 ml phosphoric acid in 1000 ml water).

N^{DISACC}-(4-(4-chlorophenyl)benzyl)A82846B may be used per se or in the form of its pharmaceutically acceptable salt, hydrate, solvate or mixtures thereof. The term "pharmaceutically acceptable salt" refers to non-toxic acid addition salts derived from inorganic and organic acids. Acids commonly employed to form acid addition salts are inorganic acids such as hydrochloric acid, hydrobromic acid, hydroidic acid, sulfuric acid, phosphoric acid, and the like, and organic acids such as *p*-toluenesulfonic acid, methanesulfonic acid, oxalic acid, *p*-bromophenylsulfonic acid, carbonic acid, succinic acid, citric acid, benzoic acid, acetic acid, and the like. Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counter-ion forming a part of. any salt of this invention is not of a critical nature, so long as the salt as a whole is pharmacologically acceptable and as long.as the counter-ion does not contribute undesired qualities to the salt as a whole.

Typically said monthly dose is for administration orally or for parenteral administration

A typical solution formulation is prepared by mixing N^{DISACC}- (4-(4-chlorophenyl)benzyl)A82846B and a surfactant in a solvent. The formulation may optionally include one or more of a buffer, a stabilizing agent, and/or a tonicity agent, Solvents are generally selected based on solvents recognized by persons in the art as safe (GRAS) to be administered parenterally to a mammal. In general, safe solvents are non-toxic aqueous solvents such as, water and other non-toxic solvents that are soluble or miscible in water. Suitable aqueous solvents include water, ethanol, propylene glycol, polyethylene glycols (e.g., PEG400, PEG300), etc. and mixtures thereof. A preferred solvent is water.

The term "tonicity agents refers to a pharmaceutically acceptable excipient that makes the solution compatible with blood. Tonicity agents are.particularly desirable in injectable formulations.

The active ingredient is typically formulated into pharmaceutical dosage forms to provide an easily controllable dosage of the drug and to give the patient an elegant and ease to handle product. When a unit dose is administered orally or parenterally, it is typically provided in the form of a tablet, capsule, pill, powder packet, topical composition, suppository, wafer, measured units in ampoules or in multidose containers, etc. The dosage to be administered may vary depending upon the physical characteristics of the patient, the severity of the patient's symptoms, and the means used to administer the drug. The specific dose for a given patient is usually set by the judgment of the attending physician. In general, an effective dose will be greater than or equal to 0.5 mg/kg body weight, preferably from 0.5 to 10 mg/kg, more preferably from 0.5 to 5 mg/kg; even more preferably 0.5 to 3 mg/kg, most preferably from 0.5 to 2.5 mg/kg. In some situations, a dose less than 0.5 mg/kg body weight per month may be effective. Although a monthly frequency is generally specified, longer time intervals between administration of the drug is clearly permissible depending upon the dose level provided'and the patient's response to the drug. Suitable frequencies include monthly, every 4-6 weeks and bimonthly.

Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which the active ingredient is being applied. The formulations may also include wetting agents, lubricating agents, emulsifiers, suspending agents, preservatives, sweeteners, perfuming agents, flavoring agents and combinations thereof.

A pharmaceutical composition may be administered using a variety of methods. Suitable methods include topical (e.g., ointments or sprays), oral, injection (e.g., intramuscular, intravenous, and intrathecal routes) and inhalation.

## Claims

1. Use of N^{DISACC}-(4-(4-chlorophenyl)benzyl)A82846B or teicoplanin, or pharmaceutically acceptable salts, hydrates or solvates thereof, or mixtures thereof, in the manufacture of a medicament for the long-term prevention of *S. pneumoniae* infection in susceptible individuals.

2. Use according to claim 1, wherein the medicament is for the periodic administration of an effective dose of N^{DISACC}-(4-(4-chlorophenyl)benzyl)A82846B or teicoplanin, or pharmaceutically acceptable salts, hydrates or solvates thereof, or mixtures thereof, wherein the time interval between successive administrations is at least one month.

3. Use according to claim 2, wherein said monthly dose is for administration orally.

4. Use according to claim 2, wherein said monthly dose is for parenteral administration.

5. Use according to claim 1 or claim 2, wherein the medicament is for the administration of N^{DISACC}- (4-(4-chlorophenyl)benzyl)A82846B or teicoplanin, or pharmaceutically acceptable salts, hydrates or solvates thereof, or mixtures thereof in a dosage of greater than or equal to 0.5 body weight.

6. Use according to claim 1 or claim 2, wherein the medicament is for the administration of N^{DISACC}-(4-(4-chlorophenyl)benzyl)A82846B or teicoplanin, or pharmaceutically acceptable salts, hydrates or solvates thereof, or mixtures thereof in a dosage from 0.5 mg/kg body weight to 10 mg/kg body weight.

7. Use according to claim 1 or claim 2, wherein. the medicament is for the administration of N^{DISACC}-(4-(4-chlorophenyl)-benzyl)A82896B or teicoplanin, or pharmaceutically acceptable salts, hydrates or solvates thereof, or mixtures thereof in a dosage from 0.5 mg/kg body weight to 5 mg/kg body weight.

8. Use according to claim 1 or claim 2, wherein the medicament is for the administration of N^{DISACC}-(4-(4-chlorophenyl)benzyl)A82846B or teicoplanin, or pharmaceutically acceptable salts, hydrates or solvates thereof, or mixtures thereof in a dosage from 0.5 mg/kg body weight to 3 mg/kg body weight.

9. Use according to claim 1 or claim 2, wherein. the medicament is for the administration of N^{DISACC}-(4-(4-chlorophenyl)benzyl)A82846B or teicoplanin, or pharmaceutically acceptable salts, hydrates or solvates thereof, or mixtures in a dosage from 0.5 mg/kg body weight to 2.5 mg/kg body weight.

10. Use according to any one of the preceding claims, wherein the medicament is in the form of a composition comprising one or more carrier, diluent or excipient.

## Patentansprüche

1. Verwendung von N^{DISACC}-(4-(4-Chlorphenyl)benzyl)A82846B oder Teicoplanin oder pharmazeutisch annehmbarer Salze, Hydrate oder Solvate hierven oder Mischungen hiervon bei der Herstellung eines Arzneimittels zur Langzeitprävention vor einer Infektion durch S. pneumoniae in suszeptiblen Individuen.

2. Verwendung nach Anspruch 1, worin das Arzneimittel für die periodische Verabreichung einer wirksamen Dosis von N^{DISACC}-(4-(4-Chlorphenyl)benzyl)A82846B oder Teicoplanin oder pharmazeutisch annehmbarer Salze, Hydrate oder Solvate hiervon oder Mischungen hiervon ist, worin das Zeitintervall zwischen sukzessiven Verabreichungen wenigstens einen Monat beträgt.

3. Verwendung nach Anspruch 2, worin diese Monatsdosis für eine orate Verabreichung ist.

4. Verwendung nach Anspruch 2, worin die Monatsdosis für eine parenterale Verabreichung ist.

5. Verwendung nach Anspruch 1 oder 2, worin das Arzneimittel zur Verabreichung von N^{DISACC}-(4-(4-Chlorphenyl)benzyl)A82846B oder Teicoplanin oder pharmazeutisch annehmbarer Salze, Hydrate oder Solvate hiervon oder Mischungen hiervon in einer Dosis ist, welche größer ist als oder gleich wie 0,5 mg/kg Körpergewicht.

6. Verwendung nach Anspruch 1 oder 2, worin das Arzneimittel zur Verabreichung von N^{DISACC}-(4-(4-Chlorphenyl)benzyl)A82846B. oder Teicoplanin oder pharmazeutisch annehmbarer Salze, Hydrate oder Solvate hiervon oder Mischungen hiervon in einer Dosis von 0,5 mg/kg Körpergewicht bis 10 mg/kg Körpergewicht ist.

7. Verwendung nach Anspruch 1 oder 2, worin das Arzneimittel zur Verabreichung von N^{DISACC}-(4-(4-Chlorphenyl)benzyl)A82846B oder Teicoplanin oder pharmazeutisch annehmbarer Salze, Hydrate oder Solvate hiervon oder Mischungen hiervon in einer Dosis von 0,5 mg/kg Körpergewicht bis 5 mg/kg Körpergewicht ist.

8. Verwendung nach Anspruch 1 oder 2, worin das Arzneimittel zur Verabreichung von N^{DISACC}-(4-(4-Chlorphenyl)benzyl)A82846B oder Teicoplanin oder pharmazeutisch annehmbarer Salze, Hydrate oder Solvate hiervon oder Mischungen hiervon in einer Dosis von 0,5 mg/kg Körpergewicht bis 3 mg/kg Körpergewicht ist.

9. Verwendung nach Anspruch 1 oder 2, worin das Arzneimittel zur Verabreichung von N^{DISACC}-(4-(4-Chlorphenyl)benzyl)A82846B oder Teicoplanin oder pharmazeutisch annehmbarer Salze, Hydrate oder Solvate hiervon oder Mischungen hiervon in einer Dosis von 0,5 mg/kg Körpergewicht bis 2,5 mg/kg Körpergewicht ist.

10. Verwendung nach einem der vorhergehenden Ansprüche, worin das Arzneimittel in der Form einer Zusammensetzung vorliegt, die ein oder mehr Träger, Verdünnungsmittel oder Hilfsstoffe enthält.

## Revendications

1. Utilisation du N^{DISACC}-(4-(4-chlorophényl) benzyl) A82846B ou de la téicoplanine, ou de sels, hydrates ou solvates pharmaceutiquement acceptables de ceux-ci, ou de mélanges de ceux-ci, pour la fabrication d'un médicament destiné à la prévention à long terme de l'infection par *S. pneumoniae* chez les individus sensibles.

2. Utilisation selon la revendication 1, dans laquelle le médicament sert à administrer de manière périodique une dose efficace de N^{DISACC}-(4-(4-chlorophényl) benzyl) A82846B ou de téicoplanine, ou de sels, hydrates ou solvates pharmaceutiquement acceptables de ceux-ci, ou de mélanges de ceux-ci, dans laquelle l'intervalle de temps entre les administrations successives est d'au moins un mois.

3. Utilisation selon la revendication 2, dans laquelle ladite dose mensuelle est destinée à une administration orale.

4. Utilisation selon la revendication 2, dans laquelle ladite dose mensuelle est destinée à une administration parentérale.

5. Utilisation selon la revendication 1 ou la revendication 2, dans laquelle le médicament sert à administrer le N^{DISACC}-(4-(4-chlorophényl) benzyl) A82846B ou la téicoplanine, ou des sels, hydrates ou solvates pharmaceutiquement acceptables de ceux-ci, ou des mélanges de ceux-ci, dans une posologie supérieure ou égale à 0,5 mg/kg de poids corporel.

6. Utilisation selon la revendication 1 ou la revendication 2, dans laquelle le médicament sert à administrer le N^{DISACC}-(4-(4-chlorophényl) benzyl) A82846B ou la téicoplanine, ou des sels, hydrates ou solvates pharmaceutiquement acceptables de ceux-ci, ou des mélanges de ceux-ci, dans une posologie comprise entre 0,5 mg/kg de poids corporel et 10 mg/kg de poids corporel.

7. Utilisation selon la revendication 1 ou la revendication 2, dans laquelle le médicament sert à administrer le N^{DISACC}-(4-(4-chlorophényl) benzyl) A82846B ou la téicoplanine, ou des sels, hydrates ou solvates pharmaceutiquement acceptables de ceux-ci, ou des mélanges de ceux-ci, dans une posologie comprise entre 0,5 mg/kg de poids corporel et 5 mglkg de poids corporel.

8. Utilisation selon la revendication 1 ou la revendication 2, dans laquelle le médicament sert à administrer le N^{DISACC}-(4-(4-chlorophényl) benzyl) A828468 ou la téicoplanine, ou des sels, hydrates ou solvates pharmaceutiquement acceptables de ceux-ci, ou des mélanges de ceux-ci, dans une posologie comprise entre 0,5 mg/kg de poids corporel et 3 mg/kg de poids corporel.

9. Utilisation selon la revendication 1 ou la revendication 2, dans laquelle le médicament sert à administrer le N^{DISACC}-(4-(4-chlorophényl) benzyl) A82846B ou la téicoplanine, ou des sels, hydrates ou solvates pharmaceutiquement acceptables de ceux-ci, ou des mélanges de ceux-ci, dans une posologie comprise entre 0,5 mg/kg de poids corporel et 2,5 mg/kg de poids corporel.

10. Utilisation selon l'une quelconque des revendications précédentes, dans laquelle le médicament se présente sous la forme d'une composition comprenant un ou plusieurs support(s), diluant(s) ou excipient(s).
